# EUROPÄISCHE PATENTANMELDUNG

(11) **EP 0 548 671 A2**
(43) Veröffentlichungstag der Anmeldung: **30.06.1993**
(21) Anmeldenummer: 92120945.8
(22) Anmeldetag: 09.12.1992
(51) Int. Cl.: C07C 59/54, A61K 31/19, C07D 263/04

(54) **Verfahren zur Herstellung von optisch aktiven alpha-Hydroxycarbonsäuren**

(30) Priorität: 20.12.1991 DE 4142190
(71) Anmelder: BAYER AG, 51368 Leverkusen (DE)
(72) Erfinder: Mohrs, Klaus-Helmut, Dr., W-5600 Wuppertal (DE); Carstens, Axel, W-2300 Kiel (DE); Hoppe, Dieter, Prof. Dr., W-2300 Kiel (DE)

(57) **Zusammenfassung**

Die Erfindung betrifft ein Verfahren zur Herstellung von optisch aktiven α-Hydroxycarbonsäuren, dadurch gekennzeichnet, daß man zunächst Carbamate in Anwesenheit einer selektiven Base und eines chelatbildenden Diamins zu einer Carbanion-Komplexverbindung umsetzt, dann dia- bzw. enantioselektiv deprotoniert und dann mit CO₂ substituiert und im letzten Schritt die heterocyclische Schutzgruppe abspaltet.

## Beschreibung

Die Erfindung betrifft ein neues Verfahren zur Herstellung von teilweise bekannten optisch aktiven α-Hydroxycarbonsäuren, die wichtige Zwischenprodukte zur Synthese von pharmazeutischen Wirkstoffen, insbesondere zur Synthese von Lipoxygenasehemmern sind.

α-Hydroxycarbonsäuren sind aus vielen Publikationen bekannt [vgl. z.B. J. Org. Chem. 24 (1957), 1301, 1308; Pat.-No. 13 52 332 (1964)].

Außerdem ist bekannt, daß die Verfahren zu ihrer Herstellung aufgrund des Einsatzes von großen Mengen an Basen, der schlechten Abspaltbarkeit von Schutzgruppen und der damit verbundenen geringen Ausbeute große Nachteile zeigen.

Die vorliegende Erfindung betrifft jetzt ein Verfahren zur Herstellung von optisch aktiven α-Hydroxycarbonsäuren der allgemeinen Formel (I) 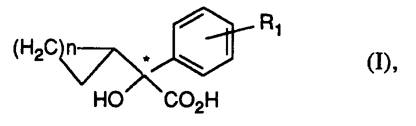
in welcher
- n: für eine Zahl 1, 2, 3, 4, 5 oder 6 steht
und
- R¹: für Wasserstoff, Halogen oder geradkettiges oder verzweigtes Alkyl mit bis zu 6 Kohlenstoffatomen oder für einen Rest der Formel -O-T steht,
worin
- T: geradkettiges oder verzweigtes Alkyl mit bis zu 6 Kohlenstoffatomen oder eine typische Hydroxyschutzgruppe bedeutet,
dadurch gekennzeichnet, daß man
Carbamate der allgemeinen Formel (II) 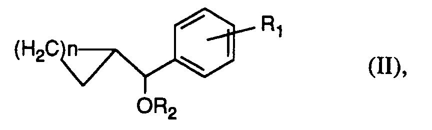
in welcher
- n und R¹: die oben angegebene Bedeutung haben,
und
- R²: für einen Rest der Formel 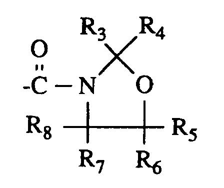 steht,
worin
- R³, R⁴, R⁵, R⁶, R⁷ und R⁸: gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten oder jeweils R³ und R⁴, R⁵ und R⁶ und/oder R⁷ und R⁸ gemeinsam einen 3- bis 6-gliedrigen, gesättigten Carbocyclus bilden,
zunächst in inerten Lösemitteln, in Anwesenheit einer selektiven Base, vorzugsweise sec.-Butyllithium, und eines chelatbildenden Diamins (im folgenden mit D bezeichnet) zu den Carbanion-Komplexverbindungen der allgemeinen Formel (III) 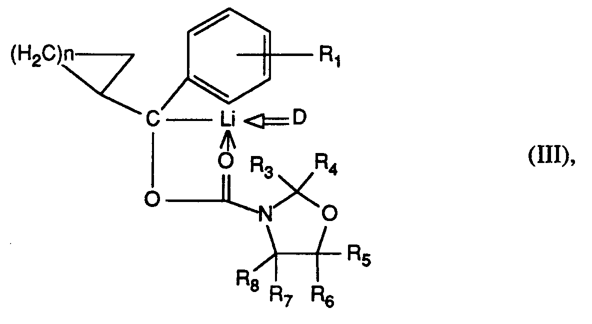
in welcher
- n, R¹, R³, R⁴, R⁵, R⁶, R⁷ und R⁸: die oben angegebene Bedeutung haben,
und
- D: für chelatbildende Diamine, wie beispielsweise (-)-Spartein oder TMEDA, steht,
dia- bzw. enantioselektiv deprotoniert und anschließend direkt durch elektrophile Substitution mit CO₂ in inerten Lösemitteln in die Verbindungen der allgemeinen Formel (IV) 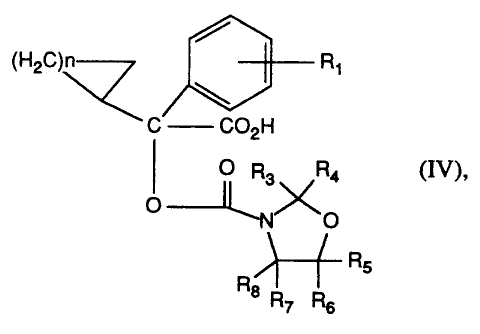
in welcher
- n, R¹, R³, R⁴, R⁵, R⁶, R⁷ und R⁸: die oben angegebene Bedeutung haben,
oder diese zunächst mit Diazomethan in Ether in die Verbindungen der allgemeinen Formel (V) 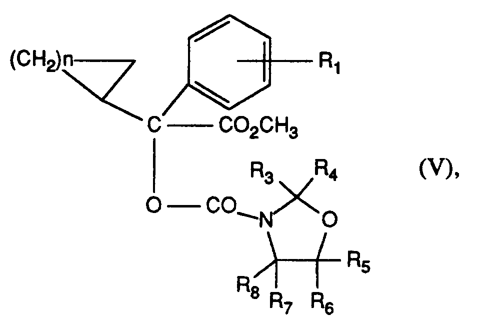
in welcher
- n, R¹, R³, R⁴, R⁵, R⁶, R⁷ und R⁸: die oben angegebene Bedeutung haben,
überführt und in einem letzten Schritt die heterocyclische Schutzgruppe nach üblichen Methoden abspaltet und die Methylreste nach üblicher Methode verseift.

Das erfindungsgemäße Verfahren kann durch folgendes Formelschema beispielhaft erläutert werden: 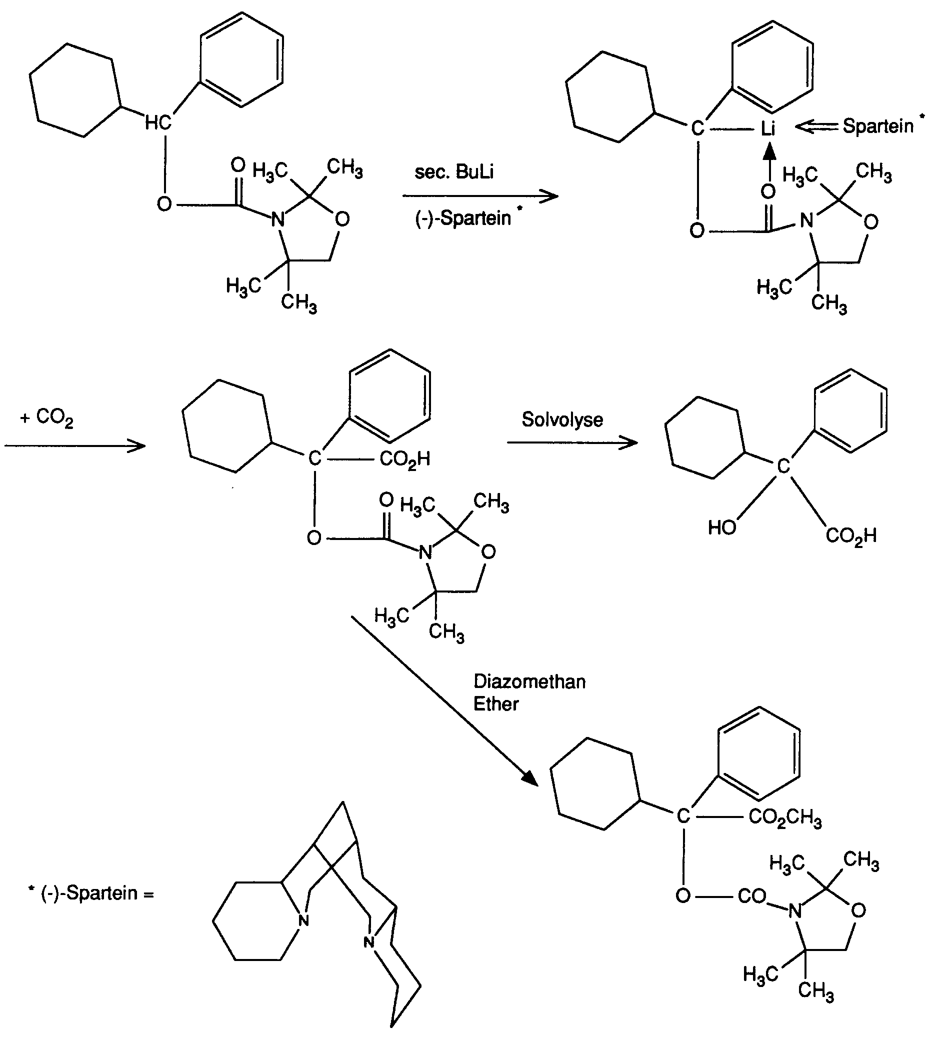
Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyl-dimethylsilyl, tert.-Butyldiphenylsilyl, Triphenylsilyl, Trimethylsilylethoxycarbonyl, Methoxymethyl oder Benzoyl. Bevorzugt sind Benzoyl, Trimethylsilyl oder tert.Butyl-dimethylsilyl.

Bevorzugt werden nach dem erfindungsgemäßen Verfahren Verbindungen der allgemeinen Formel (I),
in welcher
- n: für eine Zahl 1, 2, 3, 4, 5 oder 6 steht,
und
- R¹: für Wasserstoff, Fluor, Chlor oder geradkettiges oder verzweigtes Alkyl mit bis zu 4 Kohlenstoffatomen oder für einen Rest der Formel -O-T steht,
worin
- T: geradkettiges oder verzweigtes Alkyl mit bis zu 4 Kohlenstoffatomen, Benzoyl, Trimethylsilyl oder tert.Butyldimethylsilyl bedeutet,
hergestellt.

Besonders bevorzugt werden nach dem erfindungsgemäßen Verfahren Verbindungen der allgemeinen Formel (I),
in welcher
- n: für eine Zahl 1, 2, 3, 4, 5 oder 6 steht,
und
- R¹: für Wasserstoff, Methyl, Chlor oder für einen Rest der Formel -O-T steht,
worin
- T: Methyl, Ethyl, Benzoyl, Trimethylsilyl oder tert.Butyldimethylsilyl bedeutet,
hergestellt.

Als Lösemittel für die Deprotonierung eignen sich bevorzugt inerte organische Lösemittel wie Kohlenwasserstoffe wie Hexan, Pentan, Ligroin oder Toluol, und Ether, beispielsweise Tetrahydrofuran, Diethylether, Dioxan, Dimethoxyethan, Diglyme, Triglyme oder tert.-Butylmethylether. Besonders bevorzugt sind Tetrahydrofuran und Diethylether.

Die Deprotonierung erfolgt in einem Temperaturbereich von -100°C bis Raumtemperatur, Vorzugsweise bei ca. -78°C bis 0°C.

Die Deprotonierung kann sowohl bei Normaldruck als auch bei erhöhtem oder erniedrigtem Druck (z.B. 0,5 bis 2 bar), vorzugsweise bei Normaldruck, durchgeführt werden.

Als selektive Basen eignen sich Alkyllithiumverbindungen mit bis zu 6 C-Atomen in der Alkylgruppe, vorzugsweise n-Butyllithium oder sec.-Butyllithium.

Die Base wird in einer Menge von 0,5 bis 5 mol, vorzugsweise in stöchiometrischen Mengen, bezogen auf 1 mol der Verbindungen der allgemeinen Formel (II), eingesetzt.

Die elektrophile Substitution mit CO₂ erfolgt ebenfalls in den oben aufgeführten Lösemitteln, bevorzugt in Tetrahydrofuran, bei Normaldruck.

Die Veresterung mit Diazomethan erfolgt in Ether bei Raumtemperatur.

Die Abspaltung erfolgt nach üblicher Methode durch sequentielle Behandlung mit Säuren und Basen in einem der oben aufgeführten Lösemittel, vorzugsweise in Methanol.

Als Säuren eignen sich starke anorganische Säuren und organische Sulfon- oder Carbonsäuren wie beispielsweise Methansulfonsäure, Ethansulfonsäure, Benzolsulfonsäure, Toluolsulfonsäure, Essigsäure oder Propionsäure.

Als Basen eignen sich Alkali- und Erdalkalihydroxide wie beispielsweise Natrium-, Kalium- oder Bariumhydroxid. Bevorzugt ist Bariumhydroxid.

Die Säuren und Basen werden in einer Menge von 0,01 bis 10 mol, vorzugsweise mit 1 mol, bezogen auf 1 mol der Verbindungen der allgemeinen Formel (IV), eingesetzt.

Die Abspaltung erfolgt bei Normaldruck in einem Temperaturbereich von 0°C bis +130°C, vorzugsweise von +20°C bis +100°C.

Die Abspaltung kann auch mit Lithiumaluminiumhydrid in einem der oben aufgeführten Lösemittel, vorzugsweise Tetrahydrofuran, erfolgen.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol, bezogen auf 1 Mol des Esters, eingesetzt. Besonders bevorzugt verwendet man molare Mengen der Reaktanden.

Die Verbindungen der allgemeinen Formel (II) sind neu und können hergestellt werden, indem man

Verbindungen der allgemeinen Formel (VI) 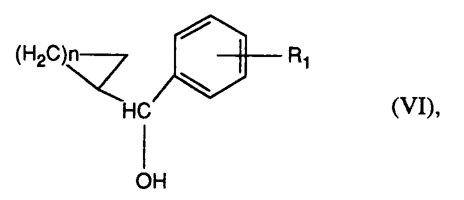
in welcher
- n und R¹: die oben angegebene Bedeutung haben,
mit Verbindungen der allgemeinen Formel (VII) 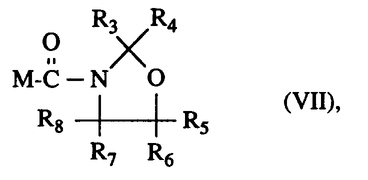
worin
- R³, R⁴, R⁵, R⁶, R⁷ und R⁸: die oben angegebene Bedeutung haben,
und
- M: für Halogen, vorzugsweise Chlor, steht,
in einem der oben aufgeführten organischen Lösemittel, vorzugsweise Diethylether, in Anwesenheit einer Base, vorzugsweise Natriumhydrid, umsetzt,
oder
Chlorameisensäurebenzylesterderivate der allgemeinen Formel (VIII) 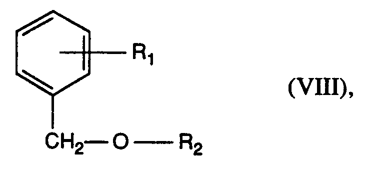
in welcher
- R¹ und R²: die oben angegebene Bedeutung haben,
zunächst in einem der oben aufgeführten Lösemittel, vorzugsweise in Tetrahydrofuran, mit TMEDA versetzt, anschließend in Hexan mit Butyllithium deprotoniert und in einem letzten Schritt mit Verbindungen der allgemeinen Formel (IX) 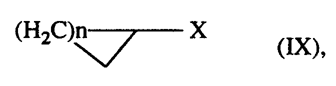
in welcher
- X: für Halogen, vorzugsweise Jod, steht,
alkyliert.

Die Deprotonierung erfolgt bei -78°C und Normaldruck.

Die Alkylierung erfolgt im Temperaturbereich von -78°C bis +40°C.

Die Verbindungen der allgemeinen Formel (VIII) sind teilweise neu und können hergestellt werden, indem man die entsprechenden Chlorameisensäurebenzylester-derivate mit den Oxazolinderivaten in Ethern in einem Temperaturbereich von -20°C bis +10°C, vorzugsweise bei 0°C und Normaldruck, umsetzt.

Die Cycloalkylhalogenide der allgemeinen Formel (IX) sind bekannt.

Die Verbindungen der allgemeinen Formeln (VI) und (VII) sind teilweise bekannt oder können nach üblichen Methoden hergestellt werden [vgl. J. Med. Chem. 31 (1), 37-54; 34 (10), 2962 - 83; PCT WO 86/07056].

Überraschenderweise liefert das erfindungsgemäße, enantioselektive Verfahren die gewünschten Verbindungen der allgemeinen Formel (I) in guten Ausbeuten.

Das Verfahren zeichnet sich durch mehrere Vorteile aus: im Gegensatz zum Stand der Technik sind stöchiometrische Mengen an Basen, vorzugsweise sec.-Butyllithium für die Deprotonierung ausreichend. Außerdem ermöglicht das erfindungsgemäße Verfahren eine Steuerung der Diastereoselektivität bei Verwendung optisch aktiver Alkohole.

Durch Einsatz von chiralem (-)-Spartein erfolgt die Deprotonierung der Verbindungen der Formel (II) enantioselektiv und in sehr guten Ausbeuten zu den entsprechenden chiralen, bevorzugt zu den (S)-Lithiumverbindungen der Formel (III), die durch weitere Umsetzung mit CO₂, bevorzugt in der R-Konfiguration überführt werden können.

Die erfindungsgemäßen Verbindungen zeichnen sich auch dadurch aus, daß nach der enantioselektiven Einführung des Elektrophils (CO₂), die Schutzgruppe R² unter Ausbildung der freien Hydroxygruppe sehr leicht abgespalten werden kann.

Die Verbindungen der allgemeinen Formeln (III), (IV) und (V) sind neu und können nach dem oben angegebenen Verfahren hergestellt werden.

Das erfindungsgemäße Verfahren ermöglicht somit einen eleganten und in guten Ausbeuten verlaufenden Zugang zu optisch aktiven α-Hydroxycarbonsäuren, die wertvolle Zwischenprodukte zur Synthese von lipoxygenasehemmenden Wirkstoffen sind [vgl. EP 344 519].

### Beispiel 1

### Benzyl-2,2,4,4-tetramethyl-oxazolidin-3-carboxylat

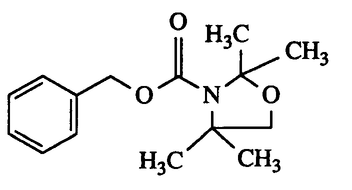

8,500 g (50,00 mmol) Chlorameisensäurebenzylester wurden in 100 ml trockenem Ether gelöst und langsam unter Eiskühlung zu einer Lösung von 12,800 g (100,00 mmol) des Oxazolidins in 100 ml trockenem Ether gegeben. Man entfernte die Eiskühlung und erhitzte noch 1 h unter Rückfluß. Nach wäßriger Aufarbeitung und Reinigung an Kieselgel (Ether/Petrolether 1:10) erhielt man 11,967 g (45,50 mmol; 91 %) der Titelverbindung.
R_{f}: 0,51 (Ether/Petrolether 1:5).

### Beispiel 2

### (Cyclohexyl-phenyl-methyl)-2,2,4,4-tetramethyl-1,3-oxazolidin-3-carboxylat

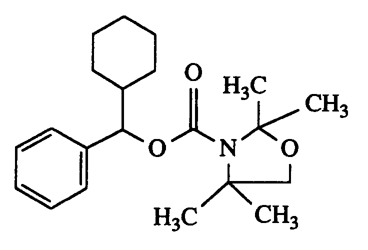

2,630 g (10,00 mmol) der Verbindung aus Beispiel 1 wurden in 10 ml trockenem THF gelöst und mit 1,160 g (10,00 mmol) TMEDA versetzt. Nachdem man auf -78°C abgekühlt hatte, deprotonierte man mit 6,3 ml (100,00 mmol) einer ca. 1,6 µ n-Butyllithium-Losung in Hexan. Nach ca. 30 min gab man 4,200 g (20 mmol) Cyclohexyliodid hinzu. Nach 4 h wurde auf Raumtemperatur aufgewärmt und wäßrig aufgearbeitet. Nach Reinigung an Kieselgel (Ether/Petrolether 1:9) erhielt man 2,736 g (7,90 mmol) 79 % der Titelverbindung.
R_{f}: 0,56 (Ether/Petrolether 1:3).

### Beispiel 3

### (Cyclooctyl-phenyl-methyl)-2,2,4,4-tetramethyl-1,3-oxazolidin-3-carboxylat

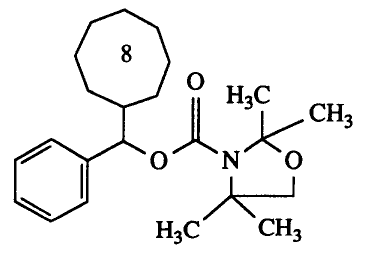

2,630 g (10,00 mmol) der Verbindung aus Beispiel 1 wurden mit 1,160 g (10,00 mmol) TMEDA in 10 ml trockenem THF gelöst und auf -78°C heruntergekühlt. Nach Zugabe von 6,3 ml (100 mmol) einer ca. 1,6 µ n-Butyllithiumlösung in Hexan ließ man noch 30 min rühren, bevor man 4,760 g (20,00 mmol) Cyclooctyliodid hineingab. Nach 5 h ließ man auf Raumtemperatur aufwärmen und arbeitete wäßrig auf. Nach Reinigung an Kieselgel (Ether/Petrolether 1:9) erhielt man 2,540 g (6,80 mmol) 68 % der Titelverbindung.
R_{f}: 0,52 (Ether/Petrolether 1:3).

### Beispiel 4

### (2-Cyclohexyl-2-phenyl)-(2,2,4,4-tetramethyl-1,5-oxazolidin-2-carbonyloxy)-essigsäuremethylester

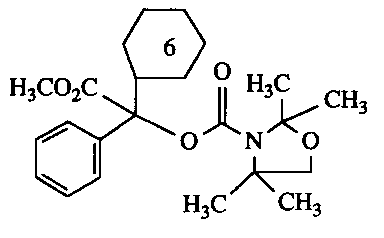

690 mg (2,00 mmol) der Verbindung aus Beispiel 2 wurden mit 235 mg (1,00 mmol) (-)-Spartein in 2 ml trockenem Ether gelöst und auf -78°C abgekühlt. Nach Zugabe von 0,73 ml (1,00 mmol) einer sec.-Butyllithiumlösung in Cyclohexan/Hexan ließ man 24 h rühren. Dann wurde für 10 min ein trockener CO₂-Strom in die Lösung eingeleitet. Nach 1 h ließ man auf Raumtemperatur aufwärmen, arbeitete sauer auf und trocknete die organische Phase über Magnesiumsulfat. Nach Filtration engte man die Lösung im Vakuum ein und addierte solange eine Lösung von Diazomethan in Ether, bis eine gelbe Färbung der Lösung bestehen blieb. Man vernichtete nach 2 h überschüssiges Diazomethan mit Kieselgel und chromatographierte den nach Einengung im Vakuum erhaltenen Rückstand an Kieselgel (Ether/Petrolether 1:6). Man erhielt neben 369 g (1,07 mmol) 53 % Produkt auch 200 mg (0,50 mmol) 25 % der Titelverbindung.
R_{f}: 0,36 (Ether/Petrolether 1:3).

### Beispiel 5

### 2-Cyclooctyl-2-phenyl-(2,2,4,4-tetramethyl-1,3-oxazolidin-3-carbonyloxy)-essigsäuremethylester

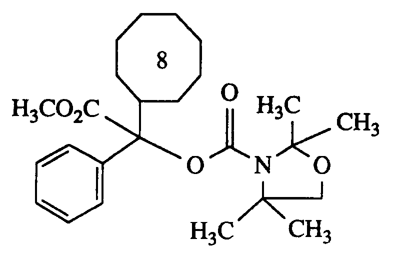

Die Herstellung erfolgt in Analogie zur Vorschrift des Beispiels 4. Man erhielt 220 mg (0,51 mmol) 25 % der Titelverbindung.
R_{f}: 0,34 (Ether/Petrolether 1:3).

### Beispiel 6

### [Cyclohexyl-(4-methoxy-phenyl)-methyl]-2,2,4,4-tetramethyl-1,3-oxazolidin-3-carboxylat

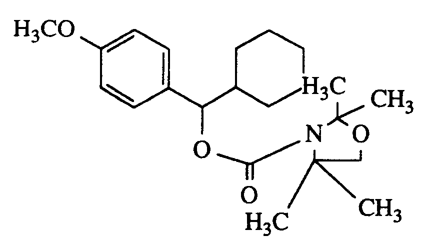

660 mg (3,00 mmol) des Alkohols wurden bei Raumtemperatur zu einer Suspension von 90,0 mg (3,00 mmol) Natriumhydrid (80 % Suspension in Paraffinöl) in 20 ml trockenem DnE gegeben. Man ließ 5 d bei Raumtemperatur rühren. Dann gab man 576 mg (3,00 mmol) des Carbamoylchlorids in DnE (10 ml) gelöst zu der Mischung und ließ weitere 5 d rühren. Nach wäßriger Aufarbeitung und Reinigung an Kieselgel (Ether/Petrolether 1:3) erhielt man 202 mg (0,54 mmol) 18 % der Titelverbindung.
R_{f}: 0,48 (Ether/Petrolether 1:5).

## Patentansprüche

1. Verfahren zur Herstellung von optisch aktiven α-Hydroxycarbonsäuren der allgemeinen Formel 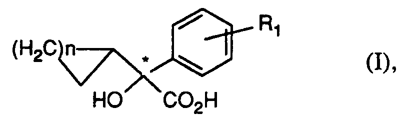 in welcher
n für eine Zahl 1, 2, 3, 4, 5 oder 6 steht
und
R¹ für Wasserstoff, Halogen oder geradkettiges oder verzweigtes Alkyl mit bis zu 6 Kohlenstoffatomen oder für einen Rest der Formel -O-T steht,
worin
T geradkettiges oder verzweigtes Alkyl mit bis zu 6 Kohlenstoffatomen oder eine typische Hydroxyschutzgruppe bedeutet,
dadurch gekennzeichnet, daß man
Carbamate der allgemeinen Formel (II) 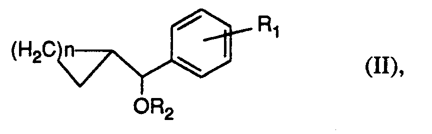 in welcher
n und R¹ die oben angegebene Bedeutung haben,
und
R² für einen Rest der Formel 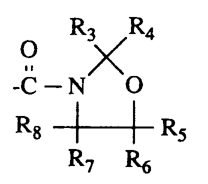 steht,
worin
R³, R⁴, R⁵, R⁶, R⁷ und R⁸ gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten oder jeweils R³ und R⁴, R⁵ und R⁶ und/oder R⁷ und R⁸ gemeinsam einen 3- bis 6-gliedrigen, gesättigten Carbocyclus bilden,
zunächst in inerten Lösemitteln, in Anwesenheit einer selektiven Base, vorzugsweise sec.-Butyllithium, und eines chelatbildenden Diamins (im folgenden mit D bezeichnet) zu den Carbanion-Komplexverbindungen der allgemeinen Formel (III) 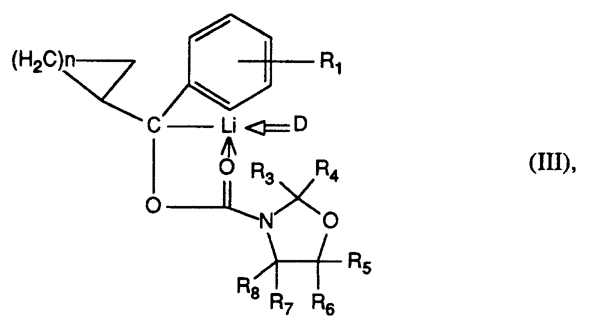 in welcher
n, R¹, R³, R⁴, R⁵, R⁶, R⁷ und R⁸ die oben angegebene Bedeutung haben
und
D für chelatbildende Diamine, wie beispielsweise (-)-Spartein oder TMEDA steht,
dia- bzw. enantioselektiv deprotoniert und anschließend direkt durch elektrophile Substitution mit CO₂ in inerten Lösemitteln in die Verbindungen der allgemeinen Formel (IV) 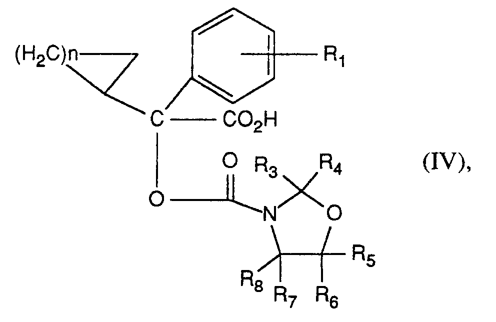 in welcher
n, R¹, R³, R⁴, R⁵, R⁶, R⁷ und R⁸ die oben angegebene Bedeutung haben,
oder diese zunächst mit Diazomethan in Ether in die Verbindungen der allgemeinen Formel (V) 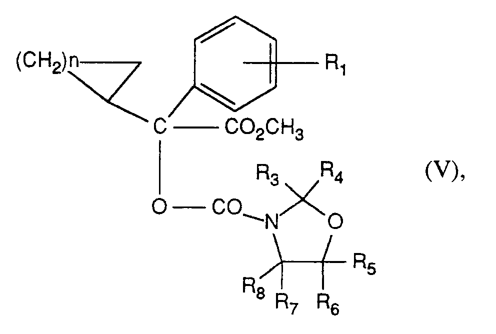 in welcher
n, R¹, R³, R⁴, R⁵, R⁶, R⁷ und R⁸ die oben angegebene Bedeutung haben,
überführt und in einem letzten Schritt die heterocyclische Schutzgruppe nach üblichen Methoden abspaltet,
und die Methylreste nach üblicher Methode verseift.

2. Verfahren nach Anspruch 1 zur Herstellung von Verbindungen der Formel (I) nach Anspruch 1, wobei
n für eine Zahl 1, 2, 3, 4, 5 oder 6 steht
und
R¹ für Wasserstoff, Fluor, Chlor oder geradkettiges oder verzweigtes Alkyl mit bis zu 4 Kohlenstoffatomen oder für einen Rest der Formel -O-T steht,
worin
T geradkettiges oder verzweigtes Alkyl mit bis zu 4 Kohlenstoffatomen, Benzoyl, Trimethylsilyl oder tert.Butyldimethylsilyl bedeutet.

3. Verfahren nach Anspruch 1 zur Herstellung von Verbindungen der Formel (I) nach Anspruch 1, wobei
n für eine Zahl 1, 2, 3, 4, 5 oder 6 steht,
und
R¹ für Wasserstoff, Methyl, Chlor oder für einen Rest der Formel -O-T steht,
worin
T Methyl, Ethyl, Benzoyl, Trimethylsilyl oder tert.Buryldimethylsilyl bedeutet.

4. Verfahren nach den Ansprüchen 1 bis 3, dadurch gekennzeichnet, daß man die Deprotonierung in einem Temperaturbereich von -100°C bis Raumtemperatur durchführt.

5. Verfahren nach den Ansprüchen 1 bis 4, dadurch gekennzeichnet, daß als selektive Base Alkyllithiumverbindungen mit bis zu 6 Kohlenstoffatomen in der Alkylgruppe eingesetzt werden.

6. Verfahren nach den Ansprüchen 1 bis 5, dadurch gekennzeichnet, daß man die Abspaltung in einem Temperaturbereich von 0°C bis +130°C durchführt.

7. Verfahren nach den Ansprüchen 1 bis 6, dadurch gekennzeichnet, daß man die Abspaltung mit Lithiumaluminiumhydrid durchführt.

8. Carbamate der allgemeinen Formel (II) 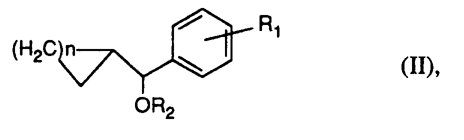 in welcher
n für eine Zahl 1, 2, 3, 4, 5 oder 6 steht,
R¹ für Wasserstoff, Halogen oder geradkettiges oder verzweigtes Alkyl mit bis zu 6 Kohlenstoffatomen oder für einen Rest der Formel -O-T steht,
worin
T geradkettiges oder verzweigtes Alkyl mit bis zu 6 Kohlenstoffatomen oder eine typische Hydroxyschutzgruppe bedeutet,
und
R² für einen Rest der Formel 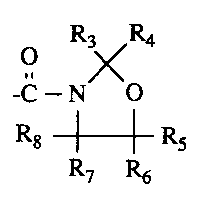 steht,
worin
R³, R⁴, R⁵, R⁶, R⁷ und R⁸ gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten oder jeweils R³ und R⁴, R⁵ und R⁶ und/oder R⁷ und R⁸ gemeinsam einen 3- bis 6-gliedrigen, gesättigten Carbocyclus bilden.

9. Carbanion-Komplexverbindungen der allgemeinen Formel (III) 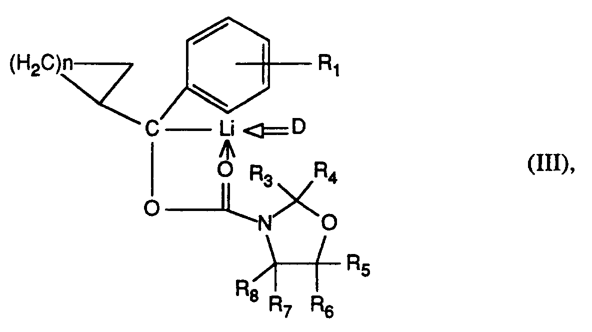 in welcher
n für eine Zahl 1, 2, 3, 4 oder 5 steht,
R¹ für Wasserstoff, Halogen oder geradkettiges oder verzweigtes Alkyl mit bis zu 6 Kohlenstoffatomen oder für einen Rest der Formel -O-T steht,
worin
T geradkettiges oder verzweigtes Alkyl mit bis zu 6 Kohlenstoffatomen oder eine typische Hydroxyschutzgruppe bedeutet,
D für chelatbildende Diamine, wie beispielsweise (-)-Spartein oder TMEDA steht,
und
R³, R⁴, R⁵, R⁶, R⁷ und R⁸ gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten oder jeweils R³ und R⁴, R⁵ und R⁶ und/oder R⁷ und R⁸ gemeinsam einen 3- bis 6-gliedrigen, gesättigten Carbocyclus bilden.

10. Verbindungen der allgemeinen Formel (IV) 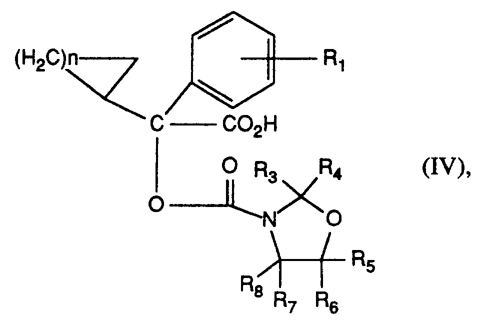 in welcher
n für eine Zahl 1, 2, 3, 4, 5 oder 6 steht,
R¹ für Wasserstoff, Halogen oder geradkettiges oder verzweigtes Alkyl mit bis zu 6 Kohlenstoffatomen oder für einen Rest der Formel -O-T steht,
worin
T geradkettiges oder verzweigtes Alkyl mit bis zu 6 Kohlenstoffatomen oder eine typische Hydroxyschutzgruppe bedeutet,
und
R³, R⁴, R⁵, R⁶, R⁷ und R⁸ gleich oder verschieden sind und Wasserstoff, geradkettiges oder verzweigtes Alkyl mit bis zu 8 Kohlenstoffatomen, Phenyl oder Cycloalkyl mit 3 bis 6 Kohlenstoffatomen bedeuten oder jeweils R³ und R⁴, R⁵ und/oder R⁶, R⁷ und R⁸ gemeinsam einen drei- bis sechsgliedrigen Carbocyclus bilden.
